# FASCICULE DE BREVET EUROPEEN

(11) **EP 0 551 418 B1**
(45) Date de publication et mention de la délivrance du brevet: **04.01.1995**
(21) Numéro de dépôt: 91919209.6
(22) Date de dépôt: 30.09.1991
(51) Int. Cl.: A61M 1/02, G01G 17/06, B01F 11/00

(54) **DISPOSITIF DE PESEE ET D'AGITATION SIMULTANEE**
VORRICHTUNG ZUM WIEGEN UND GLEICHZEITIGEN RÜHREN
SIMULTANEOUS WEIGHING AND AGITATING DEVICE

(30) Priorité: 01.10.1990 FR 9012269
(43) Date de publication de la demande: 21.07.1993
(73) Titulaire: HEMOPHARM SERVICE S.A., F-13120 Gardane (FR)
(72) Inventeur: FILLAUD, Alain, F-13100 Aix en Provence (FR)
(74) Mandataire: Moretti, René
(86) Numéro de dépôt international: FR9100763
(87) Numéro de publication internationale: WO9205812

(56) Documents cités:
- EP-A- 0 372 570
- DE-A- 3 737 304
- FR-A- 2 088 635
- FR-A- 2 511 147
- FR-A- 2 599 837
- FR-A- 2 606 639
- US-A- 3 924 700

## Description

La présente invention a pour objet un dispositif de pesée et d'agitation simultanée.

Le secteur technique de l'invention est le domaine de la construction de balance de précision électronique.

Une des applications principales de l'invention est la réalisation de dispositifs de pesée ou balance assurant une agitation simultanée du plateau de la balance recevant l'élément à peser, qui peut être une poche de prélèvement sanguin. D'autres applications sont également envisageables dans les domaines industriels nécessitant également une pesée du produit avec une assez grande précision tout en maintenant une certaine agitation de ce produit quand il s'agit de le mélanger avec un autre en cours de pesage, ce qui est peut-être le cas dans la fabrication des parfums, de médicaments, de peintures...

Dans le domaine du prélèvement sanguin, on connait déjà certains matériels qui permettent ce prélèvement en respectant les normes fixées par l'Administration telle qu'en France un maximum de 7 ml/kg du poids du donneur avec un maximum par prise de 450 ml. De plus le sang prélevé doit être mélangé à un anticoagulant qui est déjà contenu dans la poche de prélèvement.

Pour aider les opérateurs à assurer la collecte de sang dans le cadre de ces normes donc en contrôlant au mieux la quantité prise sur un donneur, divers équipements ont fait l'objet de demandes de brevet telles que :
- la demande de brevet n°2514893 déposée le 19 Octobre 1981 par la Société MCB : cette demande décrit un procédé et un dispositif de pesage programmable d'un mélange d'une suspension dont un des constituants est ajouté progressivement, avec agitation périodique des constituants ; notamment utilisé avec un mélange de sang prélevé progressivement sur un donneur et d'un anticoagulent. La caractéristique de ce dispositif est qu'après chaque période d'agitation, l'introduction du constituant extérieur est interrompue pendant chaque pesée et le dispositif comporte des moyens arrêtant les opérations alternées d'agitation d' introduction et de pesée lorsqu'une opération de pesée indique qu'un certain poids prédéterminé a été atteint.
- La demande de brevet FR 2548135 déposée le 19 Mai 1983 par la Société Vibration Internationale SARL décrit une installation pour remplissage avec vibrations de conteneurs souples sur tables de pesée. Les installations ne concernent pas le domaine des prélèvements sanguins et sont applicables à des conteneurs en forme de sac cylindrique avec valve de remplissage. Le système décrit comporte un châssis vibrant pendant ledit remplissage, et entre deux séquences de remplissage le châssis est posé sur une plate-forme de pesée située en-dessous par déplacement vertical du conteneur sur cette table de pesée.

Les inconvénients de ces deux systèmes ci-dessus sont essentiellement que la mesure du poids de liquide introduit dans la poche ou le conteneur n'est pas mesuré en continu et ne permet donc pas une bonne précision de mesure car entre deux relevés, celle-ci a pu dériver et d'autre part l'agitation étant également interrompue pendant cette mesure ne permet pas de garantir un produit homogène, ce qui dans le cas de sang prélevé peut être un inconvénient thérapeutique majeur. Ce dernier critère s'applique également à la demande de brevet d'invention n°2603190 du Centre Régional de Transfusion Sanguine déposé par Monsieur MASSE et autres, le 29 Août 1986 qui concerne un procédé d'appareil de contrôle quantitatif de prélèvement ainsi qu'un procédé et une installation d'un prélèvement pour traitement du sang d'un donneur : l'appareil comprend un bâti, un support de contenant et des moyens de mesure d'un paramètre lié à la quantité de prélèvement et divers autres moyens complémentaires. Le demandeur revendique spécifiquement le fait que le support est fixe et qu'il n'y a pas d'agitation substantielle du prélèvement dans le contenant : ceci est considéré dans la présente invention comme un inconvénient majeur comme indiqué ci-dessus.

Pour répondre à cet objectif de pesée et d'agitation simultanée, on relève d'autres demandes de brevets telles que :
- la demande de brevet n°2574540 déposé le 10 Décembre 1984 par Monsieur BRUNET et les demandes de certificats d'addition n°2544220 et 2567416 déposées le 14 Avril 1983 et 12 Juillet 1984 par Monsieur BAUDRY : ces différentes demandes concernent un système de balance associé à un agitateur pour prélèvement sanguin. Ces trois systèmes ne sont pas en fait de véritables balances puisqu'il s'agit essentiellement de système à compensation par ressort qui permet un tarage à une valeur déterminée : lorsque le plateau de la balance recevant le prélèvement considéré atteint ce niveau préalable de consigne, un mécanisme à levier arrête l'agitateur et ledit prélèvement. De plus le système, qualifié important de balance, car il n'y a pas effectivement de prise de mesure, est un système dans les trois cas considérés A base de ressort et de lame de ressort qui comme l'indiquent les inventeurs eux-mêmes ne permet une précision que de l'ordre de 50 gr. pour les 450 à 600 gr. du prélèvement. Ceci constitue une précision très large difficilement compatible avec les çaractéristiques des centrifugeuses dans lequel le sang doit être ensuite traité pour pouvoir être exploité médicalement.
- Les demandes de brevets japonais JP n°176584 du 19 Novembre 1986 et 74240 du 30 Mars 1987 de la Société Kabushiki Kaisha concernent un appareil et un procédé pour collecter des quantités constantes de sang auprès de donneurs individuels. Les divers dispositifs décrits sont parmi les plus élaborés à ce jour, car ils permettent effectivement la pesée et l'agitation simultanée de la poche de sang posée sur le dispositif concerné grâce à une mesure piézo électrique accouplée au plateau de pesée : l'ensemble du plateau et de la cellule est monté de concert sur un axe horizontal fixe permettant de basculer par des moyens de secouage l'ensemble du système. Ceci a pour inconvénient de ne pouvoir faire des mesures de pesée exacte que lors de chaque oscillation, lorsque l'on revient à un même point initial, soit toutes les deux ou trois secondes seulement. Si la mesure est faite d'une façon permanente cela induit des erreurs de mesure de l'ordre de 3 % de la pesée même s'il est effectué une correction, du fait de la déviation angulaire du berceau.
- La demande de brevet FR-2511147 de la société SODETEM déposée le 6 Août 1981 décrit un mécanisme d'agitation permettant de commander un mouvement d'oscillation d'un plateau portant la poche de prélèvement sanguin et comportant un levier relié à un moteur électrique qui permet par une bielle articulée d'assurer une telle vibration, alors que un axe de pesée porte le seul plateau qui est articulé par ailleurs à un balancier, lequel axe de pesée s'appuie sur un ressort posé sur une pièce fixe et ne permet de peser que le plateau avec l'objet posé dessus. Du fait alors de l'oscillation transmise à ce plateau par un moyen externe, comme dans les demandes de brevet japonaises ci-dessus, ceci induit des erreurs de mesure.

Tous ces systèmes de pesée avec ou sans agitation simultanée sont donc soit très rustiques et ne permettent pas une grande précision de mesure du fait de choix des moyens techniques, soit ne permettent pas d'agitation simultanée, soit sont sans agitation du tout, soit enfin ne sont pas très précis même avec un choix de techniques élaborées. Ceci est très préjudiciable car si on dépasse les normes, les conséquences juridiques ou médicales en ce qui concerne par exemple les prélèvements sanguins, peuvent être graves et si on se maintient trop en-dessous par sécurité cela coûterait très cher aux opérateurs compte tenu du coût unitaire très élevé d'une poche de prélèvement dans le cas du sang ; de plus dans ce domaine, celui-ci doit être traité ensuite par centrifugation et cette opération nécessite une précision meilleure que les 10 % de la plupart des appareils actuels. Par ailleurs, certains ne fournissent pas à l'opérateur, outre les fonctions de pesage et d'agitation simultanée, toutes les fonctions spécifiques au prélèvement sanguin telles que le clampage de la tubulure en fin de prélèvement avec un contrôle automatique, et une programmation à la demande.

Le problème posé est de réaliser une pesée, avec une agitation simultanée, d'un objet, qui peut être une poche contenant des liquides dont l'un est introduit en continu, tel que le sang d'un donneur; laquelle pesée devant être faite avec une précision compatible avec des conditions de poids prédéterminée et des contraintes de tolérance en fonction de l'usage souhaité.

Un autre objectif est de pouvoir contrôler la quantité de liquide transférée dans la poche en cours de pesage afin de stopper le transfert à une valeur prédéterminée en fonction d'une part de conditions pré-établies et d'autre part de critères vérifiés et mesurés sur place, et d'informer les opérateurs de toutes les données propres à l'opération réalisée.

Enfin un autre objectif est d'assurer l'arrêt de cette opération de pesage-agitation-transfert d'une manière automatique et certaine, lorsque les conditions voulues sont atteintes.

Une solution au problème posé est un dispositif de pesée et d'agitation simultanée comprenant un plateau pour recevoir un objet à peser, lequel objet est un récipient recevant progressivement un produit, et le plateau est associé d'une part à un moyen permettant de mesurer le poids dudit objet et d'autre part à un moyen lui transmettant un mouvement d'agitation lequel moyen d'agitation comprend un moteur électrique monté en porte à faux par rapport à l'axe (XX') de pesee verticale du plateau et entraînant en rotation une extrémité d'un bras de levier permettant le balancement d'un barreau d'agitation monté sur un axe horizontal et libre en rotation supportant ledit plateau. Un tel dispositif est connu de FR-A-2 511 147.

Suivant l'invention, le dispositif de pesee est caractérisé en ce que le plateau et le moyen d'agitation sont montés sur un seul support sur lequel est effectué alors la mesure de poids par ledit moyen qui mesure, suivant l'axe (XX'), le poids de l'ensemble constitué du plateau, de l'objet, des moyens d'agitation, du support et de tous les éléments de liaison entre-eux, lequel moyen est choisi pour effectuer ladite mesure avec une précision égale à au plus 5 % du poids de l'ensemble du dispositif et de l'objet à peser pendant l'agitation: cette précision étant préférentielle égale à au plus 1 %.

Un autre objectif de l'invention est atteint grâce à un dispositif de pesée et d'agitation simultanée suivant le descriptif ci-dessus, utilisé pour peser une poche de prélèvement de sang remplissant celle-ci progressivement à travers une tubulure, lequel dispositif comportant un système de clampage de ladite tubulure pour écraser et fermer celle-ci en fin de prélèvement.

Un autre objectif de la présente invention est également obtenu par un procédé de pesée et d'agitation simultanée pour le prélèvement sanguin grâce à un dispositif de pesee selon l'invention d'agitation, caractérise en ce que :
. on positionne ladite poche sur ledit plateau et on place la tubulure d'amener du sang dans un dispositif de clampage que l'on amène en situation de blocage grâce à un détecteur de présence de cette tubulure ;
. on programme sur un clavier relié à un micro-processeur de commande du dispositif, le volume de sang à prélever en fonction de divers paramètres tels que par exemple soit le volume de ladite poche, soit le poids du donneur respectant les normes imposées, soit par programmation directe du volume à prélever quel que soit le poids et le nombre de poches, soit par plusieurs de ces conditions ;
. on pèse en permanence l'ensemble des moyens d'agitation, du plateau, de la poche et du sang, qui sont supportés sur un même support venant en appui sur ledit système de mesure ;
. on agite simultanément et en permanence ledit plateau pendant toute la durée de l'opération du prélèvement ;
. on arrête par fermeture du dispositif de clampage le prélèvement lorsque le volume du sang correspondant au poids mesuré et contenu dans la poche a atteint celui correspondant au volume programmé initialement ;
. on affiche et on informe par tout moyen l'opérateur de la fin de l'opération et de toute information intéressant ladite opération.

Le résultat est de nouveaux dispositifs de pesée et d'agitation simultanée et un nouveau procédé utilisant ledits dispositifs spécialement adaptés au prélèvement sanguin : ceux-ci permettent de satisfaire les exigences des opérateurs grâce essentiellement à la possibilité offerte par le système d'obtenir une grande précision de mesure qui peut être de 1‰ du poids de l'objet à peser même en cours d'agitation, alors que même 1 % serait suffisant pour cette application considérée.

En effet, la conception des matériels actuels tels que décrits précédemment ne permet pas une telle précision et celle-ci peut être obtenue dans la présente invention essentiellement parce que la mesure et la pesée portent sur l'ensemble du dispositif incluant le système d'agitation : en filtrant ensuite la mesure relevée, cette agitation n'induit pas d'erreurs de mesure liées, comme dans les autres systèmes, au déplacement en rotation et en vibration des masses et des capteurs eux-mêmes mis en mouvement par le système d'agitation. De plus le choix des techniques et de montages des systèmes d'agitation et de mesures conformément au descriptif ci-après assurent et complètent la caractéristique ci-dessus pour le résultat souhaité. Pour des raisons de qualité d'homogénéité du mélange en particulier nécessaires pour les prélèvements sanguins, l'objectif est en effet d'agiter l'objet à peser simultanément pendant la pesée, sans interruption même de quelques fractions de secondes.

Un autre intérêt de la présente invention est de pouvoir proposer un matériel essentiellement pour le prélèvement sanguin qui intègre toutes les fonctions nécessaires à celui-ci, ce qu'aucun matériel ne propose à ce jour et qui combinées entre elles assurent une autonomie au dispositif qui sécurise l'opérateur et le libère de contraintes de contrôle, lui permettant de se consacrer à des tâches plus complexes et lui évitent de faire des erreurs. Or, il faut savoir en effet qu'un dépassement de normes peut être néfaste pour le donneur et qu'à contrario une prise de sang trop faible peut coûter cher aux centres de prélèvements qui en assurent ensuite le traitement car le coût de celui-ci est le même quel que soit le poids d'une poche, sans compter les problèmes de réglage des centrifugeuses qui nécessitent un respect d'un poids connu de chaque poche.

La possibilité également de rendre étanche le dispositif assure un meilleur confort pour l'opérateur qui ainsi peut laisser couler par inadvertance tout liquide sans dommage pour l'appareil.

On pourrait citer d'autres avantages de la présente invention mais ceux cités ci-dessus en montrent déjà suffisamment pour en démontrer la nouveauté et l'intérêt. La description, les dessins et les figures ci-après représentent un exemple de réalisation de l'invention mais n'ont aucun caractère limitatif : d'autres réalisations sont possibles à partir des revendications qui précisent la portée et l'étendue de cette invention en particulier en utilisant le dispositif de pesée et d'agitation simultanée dans d'autres applications que le prélèvement sanguin et jusqu'à des poids d'objet de plusieurs centaines de kilogrammes.

La figure 1 est une vue en coupe et de profil du système d'agitation et de peser simultanée suivant l'invention.

La figure 2 est une vue de profil du système de clampage.

La figure 3 est un schéma de fonctionnement du système électronique d'agitation et de pesée.

La figure 4 est une perspective d'un bâti intégrant l'ensemble du dispositif de l'invention.

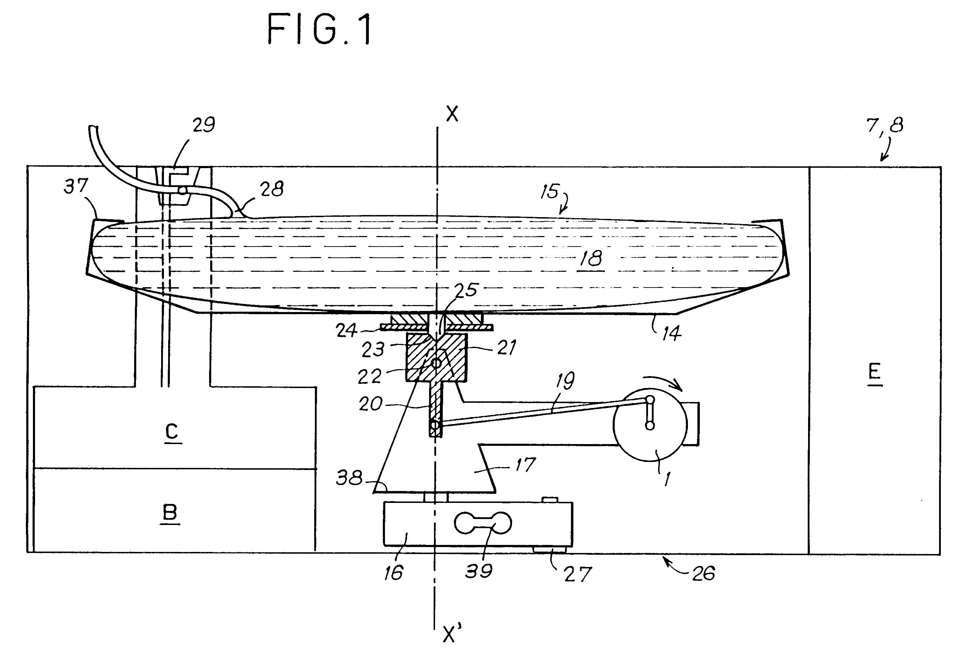

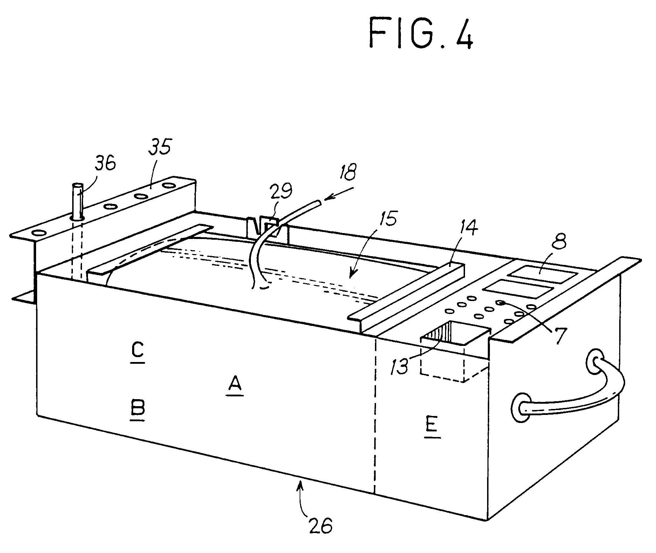

La figure 1 est une vue en coupe longitudinale simplifiée du bâti 26 représenté en perspective dans la figure 4 et dans lequel sont simplement schématisées la partie électronique et électrique E avec son clavier 7 et son écran 8 à la partie supérieure, le bloc batterie B et le système de clampage C, 29. Cette figure porte donc essentiellement sur le descriptif du système de pesée et d'agitation A.

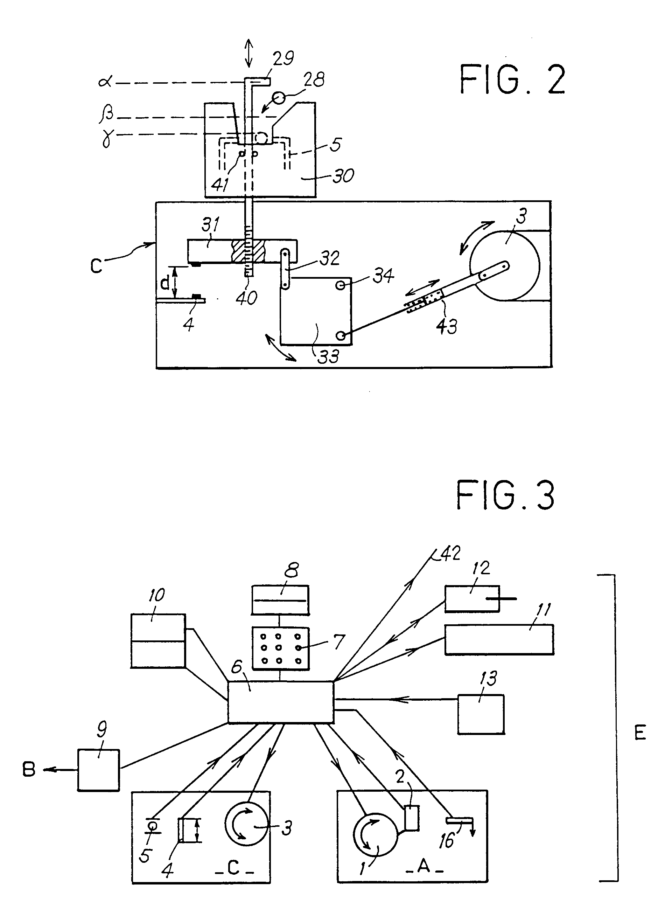

Ce système comprend un plateau 14 recevant l'objet à peser 15 : dans le présent exemple non limitatif, cet objet est une poche de prélèvement sanguin 18 comprenant une tubulure 28 passant dans la canne 29 du dispositif de clampage décrit en figure 2. Dans cet exemple, le plateau 14 est relevé à ses deux extrémités pour épouser la forme naturelle de la poche 15 et limiter l'amplitude du mouvement de liquide 18 pendant l'agitation : il peut être en métal de type aluminium, mais aussi il peut être remplacé par tout récipient tel qu'un bocal : il peut comporter aussi des rebords 37 faisant retours à chaque extrémité sur le dessus pour immobiliser lesdites poches 15. Ce plateau est directement posé sur l'ensemble du dispositif agitation et pesée qui comporte un barreau 21 d'appui supérieur, dit aussi barreau d'agitation : ce barreau peut être en acier et comporter donc un trou de centrage 23 dans l'axe xx' de pesée et ledit plateau comporte une plaque aimantée 24 et un ergot de centrage 25, laquelle plaque et lequel ergot coopèrent avec le barreau 21 et le trou 23 pour maintenir le plateau sur ledit barreau 21 et permettre de retirer le plateau et l'objet facilement.

Le moyen d'agitation est constitué essentiellement d'un moteur électrique monté sur le support 17, recevant l'ensemble des dispositifs d'agitation du plateau et de l'objet à peser, en porte à faux par rapport à l'axe XX' de pesée verticale de l'ensemble; Ce moteur 1 entraîne en rotation une extrémité d'un bras de liaison 19 relié à son autre extrémité à un levier 20 solidaire du barreau 21 d'agitation, lui-même monté sur un axe 22 horizontal, libre en rotation sur le support 17, supportant ledit plateau 14 et situé dans un plan passant par l'axe de mesure xx'.

Cette disposition permet dans un mode préférentiel de réalisation de disposer l'axe du moteur 1 dans le même plan horizontal que la fixation entre le bras de liaison 19 et le levier 20, et donc de supprimer plus facilement dans une intégration de mesure et de filtrage les composantes parasites des forces, que pourraient provoquer les mouvements de rotation et de translation. Afin d'obtenir une agitation douce et continue nécessaire pour garder l'homogénéité du produit à conserver dans la poche 15, le moteur 1 peut tourner de préférence à une vitesse de l'ordre de 10 tours par minute : il est choisi pour fournir un couple suffisant pour déplacer le levier 20 en fonction du poids des objets à supporter dans le plateau 14. Pour éviter également toute déformation et/ou vibration du support 17 qui doit assurer d'une manière rigide la fixation du moteur 1, celle du barreau 21 recevant le plateau et sa charge, et sa propre fixation sur le système de mesure de pesée 16 dans l'axe XX' auquel il transmet la totalité du poids de l'ensemble, il est nécessaire d'équilibrer dans un plan vertical la répartition des centres de gravité du moteur 1, du plateau et de sa charge ; de même il faut équilibrer le moteur 1 par rapport au système d'entraînement du levier 20 et disposer de raidisseurs sur le support 17. De préférence, celui-ci est un étrier dont la base 38 est en appui sur le système de mesure 16 et ses deux ailes latérales reçoivent d'une part à leur partie supérieur l'axe 22 du barreau d'agitation 21 situé entre elles, et d'autre part en porte à faux ledit moteur 1.

Dans un mode de réalisation préférentiel, le moyen de peser 16 est un barreau de déformation comportant une jauge de mesure, dont l'extrémité libre reçoit dans l'axe de pesée XX' le support 17 supportant l'ensemble du plateau 14, de son objet à peser 15 et du moyen d'agitation 1, et dont l'extrémité fixe 27 est solidaire du bâti 26 du dispositif. De préférence, le barreau de déformation 16 est creux en son centre 39 de part en part et comprend une jauge connue de type "binoculaire" compensée en température et calibrée permettant une pesée au gramme près jusqu'à 3000 grammes de poids : cette prise de mesure de pesée est de préférence effectuée 250 fois en une milliseconde, ce qui est assimilable à une mesure continue et donc permet ensuite un filtrage qui supprime toutes les variations cycliques éventuelles tel que celles dues aux mouvements du liquide ou à un choc ; cet échantillonnage très dense, le choix du montage et les pièces de liaison permettent d'obtenir ainsi une mesure en volume à un ml près pour un volume total de 1000 ml soit à 1 pour 1000.

La figure 2 est une vue de profil longitudinale du système de clampage C schématisé sur la figure 1 : ce système est constitué d'une canne 29 verticale recourbée à son extrémité supérieure et coulissant dans une pièce de guidage 30 échancrée, avec une ouverture en forme de V vers le haut, pour recevoir la tubulure 28; la canne 29 est fixée à sa partie inférieure à une pièce d'entraînement 31 reliée elle-même à un palonnier 33 articulé tout autour d'un axe fixe 34 et entraîné du côté opposé à ladite pièce d'entraînement 31 par une bielle 43 commandée par un moteur électrique 3.

Ce moteur électrique 3 peut être le même que celui d'agitation 1: il est réversible et peut faire un tour complet avec une commande pas à pas pour s'arrêter dans toutes positions. En effet, la canne 29 peut prendre trois positions α, β, Γ suivant son axe vertical, contrôlées par un capteur de mesure de hauteur 4 qui commande la rotation dudit moteur 3.

La position haute α correspond à une position d'ouverture pour engager ou dégager la tubulure 28, la position β intermédiaire permet de maintenir celle-ci en position sans écrasement ni déformation, et la position basse Γ pince complètement la tubulure pour empêcher toute circulation du fluide tel que le sang à l'intérieur. Ces trois positions sont détectées et contrôlées par un capteur 4 de distance qui peut être une diode optique et dont la précision est de l'ordre de 0,5 mm et mesurant la distance entre une partie fixe solidaire du bâti et la pièce d'entraînement 31 qui elle-même est réglée à une bonne hauteur par rapport à la canne 29 grâce à un filetage 40. Pour assurer que la canne se déplace verticalement, sans déformation qui fausserait la mesure, un maillon de liaison 32 relie la pièce à entraînement 31 au palonnier 34. Pour éviter d'endommager la tubulure 28 ou le système d'entraînement, la bielle 43 est une bielle à ressort pouvant absorber les différences de diamètre de ladite tubulure 28 et pour éviter les risques de dégât en cas de blocage d'une pièce rigide sous la canne 29.

Afin de rendre le dispositif, tel que représenté dans la figure 4 et schématisé dans la figure 3, autonome et fiable, le bloc fixe ou pièce de guidage 30 comprend à la base de son ouverture en V des capteurs 5 de repérage de type diode, permettant de détecter la présence ou l'absence de tubulure 28 afin d'empêcher tout fonctionnement tant que celle-ci n'est pas en place et assurer son maintien par la canne 29 dès sa mise en place : ce détecteur de présence peut également mesurer la densité optique de la tubulure et en déduire la mesure du diamètre correspondant de celle-ci, à un millimètre près par exemple, ce qui peut être ensuite intégrer dans la commande du moteur 3 et de la mesure des positions de hauteur du capteur 4 pour tenir compte justement des variations de diamètre éventuelles et éviter les écrasements lors du maintien des tubulures, en plus de la sécurité de la bielle à ressort 43.

Enfin, un joint torique 4 immobilisé dans le bloc 30 autour de la canne 29 assure une étanchéité au système contre toute fuite de liquide.

La figure 3 est un schéma de fonctionnement d'un dispositif intégrant toutes les fonctions décrites ci-dessus et éventuellement d'autres pour une application de prélèvement sanguin grâce à en particulier, un micro-processeur 6 de contrôle et de commande : ce dispositif intégré permet en particulier de suivre et d'effectuer le procédé tel que cité en présentation des présentes figures. Ainsi, le microprocesseur 6 est associé à des mémoires 10 de type "EPROM" de 8 kilo octets minimum, pour stocker le programme de gestion et de base du système et de type "RAM" de 256 kilo octets par exemple pour son fonctionnement. Un clavier 7 permet d'y entrer manuellement des données conformes au procédé et à d'autres fonctions: un afficheur 8 connu de tout type donne les informations souhaitées à l'opérateur pour qu'il puisse valider ses données, contrôler le déroulement de l'opération et obtenir visuellement les résultats. Ces données peuvent aussi être sorties sur une imprimante 11 reliée au microprocesseur 6; elle peut être intégrée éventuellement au dispositif pour permettre par exemple d'imprimer des étiquettes auto-collantes qui sont alors collées sur la poche 14 de prélèvement pour identifier les caractéristiques de celles-ci et de celles du donneur.

On peut également connecter et rajouter au système un lecteur de cartes personnalisées 12 et/ou une liaison à tous micro-ordinateurs externes 42.

Le micro-processeur assure et contrôle la charge des batteries 9, autonomes et, de préférence de 12 V, nécessaires au fonctionnement de l'ensemble, et pilote donc soit par son programme intégré, soit par commande au clavier 7 les différentes fonctions telles que :
- le système d'agitation et de pesée A avec le moteur 1 et le capteur de mesure 16 décrit précédemment ainsi qu'un capteur de position 2 du moteur pour s'assurer d'arrêter le plateau 14 dans une position toujours la même en fin de prélèvement telle qu'horizontale.
- le système de clampage C avec le moteur 3, le capteur de position 4 et de détection de présence de tubulure 5 tel que décrit précédemment,
- un ou plusieurs tests d'analyse de sang à prélever, préalablement au démarrage du prélèvement grâce à tout système connu 13 intégré au dispositif, lequel système permet de connaître les possibilités de prélèvements sanguins en relation avec les résultats biologiques de tests. Le système permet ainsi, grâce à un calcul interne au programme et en fonction de plusieurs paramètres pris en compte, de déterminer le volume net prélevable, et donc la limite maximum du volume total qui peut être prélevée en fonction du sang du donneur, ladite limite permettant alors la programmation dudit dispositif.

Cette possibilité est d'un intérêt particulier en bloc opératoire dans l'indication de l'hémodilution normale intentionnelle ainsi que dans le prédosage transfusionnel

Ces tests peuvent être essentiellement des tests d'hémoglobine et/ou d'hématocrite qui font réagir sur le sang des réactifs consommables permettant dans certains cas suivant les résultats trouvés de limiter le prélèvement en volume inférieur aux normes ou au contraire de permettre de prélever davantage si nécessaire comme lors de préparation d'opérations. L'opérateur peut alors interpréter de lui-même le volume définitif à prélever ou faire confiance au dispositif si le micro-processeur dispose d'un programme correspondant et associé.

La figure 4 représente une vue perspective de l'ensemble du dispositif comportant un bâti 26 en forme de boîtier étanche dans laquelle est disposé l'ensemble des systèmes de pesée et d'agitation A et de clampage C, et qui comporte en outre une partie électronique E composé au moins d'un micro-processeur 6 qui commande les différentes fonctions de pesée, d'agitation et de clampage telles que définies ci-dessus, d'un clavier 7 pour introduire les informations nécessaires au fonctionnement dudit micro-processeur, d'un affichage 8 et d'un jeu de batteries B, 9 autonomes.

Ledit bâti 26 peut comporter sur au moins un de ses côtés un ratelier 35 pouvant recevoir plusieurs tubes à essais 36 pour y stocker des échantillons du sang prélevé.

On distingue sur cette figure également la poche de prélèvement 15 et sa tubulure 18 posé sur le plateau 14, et le système de test d'analyse biologique intégré 13 représenté ici par une ouverture permettant de recevoir un tube à essai et, en dessous et autour de laquelle des capteurs permettent d'effectuer ledit test. Le bâti 26 peut être fermé par un couvercle transportable par des poignées . La partie inférieure du bâti peut recevoir sous forme de tiroirs latéraux amovibles une imprimante de type jet d'encre pour des étiquettes qui sont ensuite collées sur lesdites poches, un lecteur de cartes personnelles et d'autres fonctions complémentaires.

## Revendications

1. Dispositif de pesée et d'agitation simultanée comprenant un plateau (14) pour recevoir un objet (15) à peser, lequel objet est un récipient (15) recevant progressivement un produit (18), et le plateau (14) est associé d'une part à un moyen (16) permettant de mesurer le poids dudit objet et d'autre part à un moyen (1) lui permettant un mouvement d'agitation, lequel moyen d'agitation (1) comprend un moteur électrique monté en porte à faux par rapport à l'axe (XX') de pesée verticale du plateau (14) et entraînant en rotation une extrémité d'un bras de levier (19) permettant le balancement d'un barreau (21) d'agitation monté sur un axe (22) horizontal et libre en rotation supportant ledit plateau, caractérisé en ce que lesdits plateaux (14) et moyens d'agitation (1) sont montés sur un seul support (17) sur lequel est effectué alors la mesure de poids par ledit moyen (16) qui mesure, suivant l'axe (XX'), le poids de l'ensemble constitué du plateau (14), de l'objet (15), des moyens d'agitation (1), du support (17) et de tous les éléments de liaison entre eux, lequel moyen (16) est choisi pour effectuer ladite mesure avec une précision égale au plus à 5% du poids de l'ensemble du dispositif et de l'objet (15) à peser pendant l'agitation.

2. Dispositif de pesée et d'agitation simultanée selon la revendication 1, caractérisé en ce que lesdits moyens de mesure comporte un barreau (21) d'appui supérieur en acier et un trou de centrage (23) dans l'axe XX' de pesée et ledit plateau comporte une plaque aimantée (24) et un ergot de centrage (25), laquelle plaque et lequel ergot coopèrent avec le barreau (21) et le trou (23) pour maintenir le plateau sur ledit barreau (21) et permettre de retirer le plateau et l'objet facilement.

3. Dispositif de pesée et d'agitation simultanée selon l'une quelconque des revendications 1 et 2 caractérisé en ce que le moyen de peser (16) est un barreau de déformation comprenant une jauge de mesure, dont l'extrémité libre reçoit dans l'axe de pesée XX' le support (17) supportant l'ensemble du plateau (14), de son objet à peser (15) et du moyen d'agitation (1), et l'extrémité fixe (27) est solidaire du bâti (26) du dispositif.

4. Dispositif de pesée et d'agitation simultanée selon l'une quelconque des revendications 1 à 3 et est utilisé pour peser une poche (15) de prélèvement de sang (18) remplissant celle-ci progressivement à travers une tubulure (28) lequel dispositif comportant un système (29) de clampage de ladite tubulure pour écraser et fermer celle-ci en fin de prélèvement, caractérisé en ce que ledit système de clampage est constitué d'une canne (29) verticale recourbée à son extrémité supérieure et coulissant dans une pièce de guidage (30) échancrée, avec une ouverture en forme de V vers le haut, pour recevoir ladite tubulure (28) laquelle canne (29) est fixée à sa partie inférieure à une pièce d'entraînement (31) reliée elle-même à un palonnier (33) articulé tout autour d'un axe fixe (34) et entraîné du côté opposé à ladite pièce (31) par une bielle (43) commandée par un moteur électrique (3).

5. Dispositif de pesée et d'agitation simultanée suivant la revendication 4 caractérisé en ce que la canne (29) peut prendre trois positions (α, β, Γ) contrôlées par un capteur de mesure de hauteur (4) qui commande la rotation du moteur (3).

6. Dispositif de pesée et d'agitation simultanée suivant l'une quelconque des revendications 4 et 5 caractérisé en ce que ladite bielle (43) est une bielle à ressort pouvant absorber les différences de diamètre de ladite tubulure (28).

7. Dispositif de pesée et d'agitation simultanée suivant l'une quelconque des revendications 4 à 6 caractérisé en ce qu'il comporte un bâti (26) en forme de boîte étanche dans laquelle est disposée l'ensemble des systèmes de pesée et d'agitation (A) et de clampage (C), et qui comporte en outre une partie électronique (E) composé au moins d'un micro processeur (6) qui commande les différentes fonctions de pesée, d'agitation et de clampage, d'un clavier (7) pour introduire les informations nécessaires au fonctionnement dudit micro-processeur, d'un affichage (8) et d'un jeu de batteries (B, 9) autonomes.

8. Dispositif suivant la revendication 7 caractérisé en ce que ledit bâti (26) comporte sur au moins un de ses côtés un ratelier (35) pouvant recevoir plusieurs tubes à essais (36) pour y stocker des échantillons du sang prélevé.

9. Procédé de pesée et d'agitation simultanée pour le prélèvement sanguin grâce à un dispositif suivant l'une quelconque des revendications 1 à 8, caractérisé en ce que :
. on positionne ladite poche (15) sur ledit plateau et on place la tubulure (28) d'amener du sang dans un dispositif de clampage (29) que l'on amène en situation de blocage grâce à un détecteur (5) de présence de cette tubulure ;
. on programme sur un clavier relié à un micro-processeur (6) de commande du dispositif, le volume de sang à prélever en fonction de divers paramètres tels que par exemple le volume de ladite poche (15), le poids du donneur respectant les normes imposées ou par programmation directe du volume à prélever ;
. on pèse en permanence l'ensemble des moyens (1) d'agitation, du plateau (14), de la poche (15) et du sang (18), qui sont supportés sur un même support (17) venant en appui sur ledit système de mesure (16) ;
. on agite simultanément et en permanence ledit plateau pendant toute la durée de l'opération du prélèvement ;
. on arrête par fermeture du dispositif de clampage (29) le prélèvement lorsque le volume du sang contenu dans la poche (15) a atteint celui correspondant au volume programmé initialement ;
. on affiche et on informe par tout moyen l'opérateur de la fin de l'opération et de toute information intéressant ladite opération.

10. Procédé de pesée et d'agitation simultanée suivant la revendication 9 caractérisé en ce qu'on effectue préalablement au démarrage du prélèvement un test d'analyse biologique du sang à prélever grâce à tout système connu (13) intégré au dispositif, lequel système permet de connaître la limite maximum du volume total qui peut être prélevée en fonction du sang du donneur, ladite limite permettant alors également la programmation dudit dispositif.

## Claims

1. A device for simultaneous weighing and agitation, the deuce comprising a tray (14) for receiving an object (15) to be weighed, which object is a receptacle (15) that progressively receives a substance (18), and the tray (14) is associated firstly with means (16) enabling the weight of said object to be measured and secondly with means (1) enabling agitation motion to be imparted thereto, which agitation means (1) comprises an electric motor mounted in a cantilevered out position from the vertical weighing axis XX' of the tray (14) and rotating one end of a lever arm (19) enabling rocking motion to be imparted to an agitation bar (21) that is mounted free to rotate on a horizontal axis 22 and that supports said tray, the device being characterized in that said trays (14) and said agitation means (1) are mounted on a single support (17) on which weight measurement is then performed by said measurement means (16) which measures the combined weight on the axis (XX') constituted by the tray (14), the object (15), the agitation means (1), the support (17) and all the connection elements therebetween, which means (16) is chosen to perform said measurement with an error equal to not more than 5% of the weight of the combined weight of the device and the object (15) to be weighed during agitation.

2. A device for simultaneous weighing and agitation according to claim 1, characterized in that said measurement means include a bar (21) having a top steel thrust member and a centering hole (23) on the weighing axis (XX'), and said tray includes a magnetized plate (24) and a centering stud (25), which plate and which stud co-operate with the bar (21) and the hole (23) to hold the plate on said bar (21) and to enable the tray and the object to be withdrawn easily.

3. A device for simultaneous weighing and agitation according to claim 1 or 2, characterized in that the weighing means (16) is a deformable bar including a strain gauge, the free end of the bar receiving the support (17) on the weighing axis (XX') where it supports the assembly comprising the tray (14), the object thereon to be weighed (15) and the agitation means (1), and the stationary end (27) thereof is secured to the stand (26) of the device.

4. A device for simultaneously weighing and agitation according to any one of claims 1 to 3 and used for weighing a bag (15) for collecting blood (18) that fills it progressively via a tube (28), said device including a clamping system (29) for clamping said tube to compress it and close it on completion of withdrawing blood, the device being characterized in that said clamping system is constituted by a vertical bracket (29) curved over at its top end and sliding in a notched guide part (30) having an upwardly directed V-shaped opening for receiving said tube (28), which bracket (29) has its bottom portion fixed to a drive part (31) itself connected to a crank (33) hinged about a stationary axis (34) and driven on its side opposite to that connected to said part (31) by a connecting rod (43) controlled by an electric motor (3).

5. A device for simultaneous weighing and agitation according to claim 4, characterized in that the bracket (29) can take up three positions (α, β, γ) monitored by a height measurement sensor (4) that controls rotation of the motor (3).

6. A device for simultaneous weighing and agitation according to claim 4 or 5, characterized in that said connecting rod (43) is a spring rod capable of compensating differences in diameter of said tube (28).

7. A device for simultaneous weighing and agitation according to any one of claims 4 to 6, characterized in that it includes a stand (26) in the form of a moistureproof box in which the weighing, agitation (A) and clamping (C) systems are disposed together, and which also includes an electronics portion (E) comprising at least a microprocessor (6) that controls the various weighing, agitation, and clamping functions, a keypad (7) to receive the information required for the operation of said microprocessor, a display (8), and a set of self-contained batteries (B, 9).

8. A device according to claim 7, characterized in that said stand (26) includes a rack (35) on at least one of its sides suitable for receiving a plurality of test tubes (36) for storing samples therein of the blood being withdrawn.

9. A method for simultaneous weighing and agitation for use in withdrawing blood by means of a device according to any one of claims 1 to 8, the method being characterized in that:
said bag (15) is placed on said tray and the blood feed tube (28) is placed in the clamping device (29) which is placed in a locking situation by means of a detector (5) for detecting the presence of said tube;
a keypad connected to a controlling microprocessor (6) for the device is used for programming the volume of blood to be withdrawn as a function of various parameters, e.g. such as: the volume of said bag (15); the weight of the donor in compliance with standards laid down; or direct programming of the volume to be withdrawn;
the assembly comprising the agitation means (1), the tray (14), the bag (15) and the Blood (18) is weighed continuously, said assembly being supported on a common support (17) bearing on said measuring system (16);
said tray is simultaneously and continuously agitated throughout the entire duration of the blood-withdrawal operation;
withdrawal is stopped by closing the clamping device (29) when the volume of blood contained in the bag (15) has reached the volume which corresponds to the initially programmed volume; and
the termination of the operation and any information relating to said operation is displayed or conveyed to the operator by any means.

10. A method for simultaneous weighing and agitation according to claim 9, characterized in that prior to beginning to withdraw blood, a biological analysis test is performed on the blood to be withdrawn using any known system (13) integrated in the device, which system serves to indicate the maximum limit on the total volume that may be withdrawn as a function of the donor's blood, said limit then also making it possible to program said device.

## Patentansprüche

1. Vorrichtung zum wiegen und gleichzeitigen Rühren mit einem Teller (14), der einen zu wiegenden Gegenstand (15) aufnimmt, welcher Gegenstand ein Behältnis (15) ist, das nach und nach ein Produkt (18) aufnimmt, und der Teller (14) ist einerseits mit einer Vorrichtung (16) verbunden, mit der das Gewicht des besagten Gegenstands gewogen werden kann, und andererseits mit einer Vorrichtung (1), die ihm eine Umrührbewegung erlaubt, wobei die Umrührvorrichtung (1) einen Elektromotor aufweist, der bezüglich der vertikalen wiegeachse (XX') des Tellers (14) hervorstehend montiert ist und den Drehantrieb eines Endes eines Hebelarms (19) bewirkt, der das Schwenken eines Rührstabs (21) erlaubt, der an eine horizontal angeordnete und frei drehbare Achse (22) montiert ist, die den Teller trägt, dadurch gekennzeichnet, daß die Teller (14) und Rührvorrichtungen (1) auf einen gemeinsamen Träger (17) montiert sind, an dem dann die Gewichtsmessung durch die Vorrichtung (16) vorgenommen wird, welche entlang der Achse (XX') das Gewicht der Einheit aus Teller (14), Gegenstand (15), Rührvorrichtungen (1), Träger (17) und allen ihren Verbindungsvorrichtungen untereinander mißt, wobei die Vorrichtung (16) so gewählt ist, daß sie diese Messung mit einer Präzision vornimmt, die höchstens gleich 5 % des Gewichts der Einheit aus Vorrichtung und Gegenstand (15) ist, die während des Rührens zu wiegen sind.

2. Vorrichtung zum wiegen und gleichzeitigen Rühren nach Anspruch 1, dadurch gekennzeichnet, daß die genannten Meßvorrichtungen einen oberen Auflagestab (21) aus Stahl sowie eine Zentrieröffnung (23) auf der wiegeachse XX' aufweisen, und daß der Teller eine Magnetplatte (24) und einen Zentrierzapfen (25) aufweist, wobei die Platte und der Zapfen mit dem Stab (21) und der Öffnung (23) zusammenwirken, um den Teller auf dem Stab (21) zu halten und ein leichtes wegnehmen des Tellers und des Gegenstands zu ermöglichen.

3. Vorrichtung zum wiegen und gleichzeitigen Rühren nach einem der Ansprüche 1 und 2, dadurch gekennzeichnet, daß die Wiegevorrichtung (16) ein Deformationsstab mit einer Meßlehre ist, dessen freies Ende auf der Meßachse XX' den Träger (17) aufnimmt, der die Einheit aus Teller (14), seinem zu wiegenden Gegenstand (15) und Rührvorrichtung (1) aufnimmt, und daß das feste Ende (27) fest mit dem Rahmen (26) der Vorrichtung verbunden ist.

4. Vorrichtung zum Wiegen und gleichzeitigen Rühren nach einem der Ansprüche 1 bis 3, die zum Wiegen eines Beutels (15) zur Blutabnahme (18) benutzt wird und diesen allmählich durch einen Stutzen (28) hindurch befüllt, wobei diese Vorrichtung eine Klammervorrichtung (29) für diesen Stutzen aufweist, um diesen zur Abnahme plattzudrücken und zu schließen, dadurch gekennzeichnet, daß das Klammersystem aus einem vertikalen Rohr (29) besteht, das an seinem oberen Ende umgebogen ist und in einem bogenförmig ausgeschnittenen Führungsstück (30) gleitet, das nach oben eine V-förmige Öffnung aufweist, um den Stutzen (28) aufzunehmen, wobei dieses Rohr (29) in seinem unteren Bereich mit einem Antriebsteil (31) verbunden ist, das selbst mit einem Hebel (33) verbunden ist, der um eine feste Achse (34) schwenkt und auf der zu dem Antriebsteil (31) entgegengesetzten Seite von einer Pleuelstange (43) angetrieben wird, die über einen Elektromotor (3) gesteuert wird.

5. Vorrichtung zum Wiegen und gleichzeitigen Rühren nach Anspruch 4, dadurch gekennzeichnet, daß das Rohr (29) drei Stellungen (α, β, Γ) einnehmen kann, die von einem Höhenmeßfühler (4) überwacht werden, der die Drehung des Motors (3) steuert.

6. Vorrichtung zum Wiegen und gleichzeitigen Rühren nach einem der Ansprüche 4 und 5, dadurch gekennzeichnet, daß die Pleuelstange (43) eine Stange mit einer Feder ist, welche die Unterschiede im Durchmesser des Stutzens (28) aufzunehmen fähig ist.

7. Vorrichtung zum Wiegen und gleichzeitigen Rühren nach einem der Ansprüche 4 bis 6, dadurch gekennzeichnet, daß sie einen Rahmen (26) in Form eines dichten Kastens aufweist, in dem alle Vorrichtungen zum Wiegen und zum Rühren (A) und zum Klammern (C) untergebracht sind, und der außerdem einen Elektronikteil (E) aufweist, der mindestens aus einem Mikroprozessor (6), der die verschiedenen Wiege-, Rühr- und Klammerfunktionen steuert, einer Tastatur (7) zum Eingeben der für den Betrieb des Mikroprozessors nötigen Daten, einer Sichtanzeige (8) und einem Satz netzunabhängiger Batterien (B, 9) besteht.

8. Vorrichtung nach Anspruch 7, dadurch gekennzeichnet, daß der Rahmen (26) an mindestens einer seiner Seiten ein Gestell (35) zur Aufnahme mehrerer Reagenzgläser (36) zur Aufbewahrung von Blutproben aufweist.

9. Vorrichtung zum Wiegen und gleichzeitigen Rühren zur Blutabnahme mittels einer Vorrichtung nach einem der Ansprüche 1 bis 8, dadurch gekennzeichnet, daß:
. man den Beutel (15) auf den Teller legt und den Blutzufuhrstutzen (28) in eine Klammervorrichtung (29) einlegt, die man mittels eines Detektors (5), der das Vorhandensein dieses Stutzens feststellt, in eine Blockierstellung bringt;
. man auf der mit einem Mikroprozessor (6) für die Steuerung der Vorrichtung verbundenen Tastatur die zu entnehmende Blutmenge eingibt, in Abhängigkeit diverser Parameter, beispielsweise des Volumens des Beutels (15), des Gewichts des Spenders, unter Beachtung der vorgeschriebenen Normen, oder durch direkte Programmierung der abzunehmenden Menge;
. man die Vorrichtungen zum Rühren (1), den Teller (14), den Beutel (15) und das Blut (18), die alle von einem Träger (17) getragen werden, der auf der Wiegevorrichtung (16) aufliegt, ständig zusammen wiegt;
. man den Teller gleichzeitig und ständig während der gesamten Dauer des Abnahmevorgangs bewegt;
. man die Abnahme durch Schließen der Klammervorrichtung (29) beendet, wenn die in dem Beutel (15) enthaltene Blutmenge den anfangs programmierten Wert erreicht hat;
. der Bedienungsperson das Ende des Vorgangs angezeigt und sie darüber mittels einer beliebigen Vorrichtung informiert wird und sie mit allen diesen Vorgang betreffenden Informationen versorgt wird.

10. Verfahren zum Wiegen und gleichzeitigen Rühren nach Anspruch 9, dadurch gekennzeichnet, daß man vor Beginn der Abnahme mit einer beliebigen bekannten, in die Vorrichtung integrierten Vorrichtung (13) einen Test zur biologischen Analyse des abzunehmenden Bluts vornimmt (36), wodurch man die Menge feststellen kann, die abhängig vom Blut des Spenders höchstens entnommen werden kann, wobei dieser Grenzwert dann ebenfalls die Programmierung der Vorrichtung ermöglicht.
